(19) Europäisches Patentamt
European Patent Office
Office européen des brevets

(11) EP 1 426 445 A1

(12) EUROPÄISCHE PATENTANMELDUNG

(43) Veröffentlichungstag:
**09.06.2004 Patentblatt 2004/24**

(51) Int Cl.$^7$: **C12P 7/62**, C12P 17/06

(21) Anmeldenummer: **02292969.9**

(22) Anmeldetag: **03.12.2002**

(84) Benannte Vertragsstaaten:
**AT BE BG CH CY CZ DE DK EE ES FI FR GB GR IE IT LI LU MC NL PT SE SI SK TR**
Benannte Erstreckungsstaaten:
**AL LT LV MK RO**

(71) Anmelder: **Cognis France S.A.**
**31360 Saint-Martory (FR)**

(72) Erfinder:
- **Moussou, Philippe**
  **54000 Nancy (FR)**
- **Falcimaigne, Aude**
  **54000 Nancy (FR)**
- **Pauly, Gilles**
  **54000 Nancy (FR)**
- **Ghoul, Mohamed**
  **54000 Nancy (FR)**
- **Engasser, Jean-Marc**
  **54710 Ludres (FR)**
- **Ardhaoui, Melika**
  **3211 (C) Résidence de Velodrome**
  **54500 Vandoeuvre-les-Nancy (FR)**

(74) Vertreter: **Herrburger, Pierre et al**
**Cabinet Pierre Herrburger**
**115, boulevard Haussmann**
**75008 Paris (FR)**

# (54) Herstellung von Flavonoidderivaten

(57) Vorgeschlagen wird ein Verfahren zur enzymatischen Synthese von Flavonoidestern und - derivaten, bei dem man

a) ein Reaktionsmedium herstellt, enthaltend ein organisches Lösungsmittel, ein glycosyliertes Flavonoid oder Aglyconflavonoid, einen Acylgruppendonor und einen enzymatischen Katalysator,
b) während der Reaktion weitere Mengen an Flavonoid und/oder Acyldonor zusetzt und
c) die so erhaltenen Ester reinigt, indem man enzymatische Partikel und das Lösungsmittel abtrennt,

dadurch charakterisiert, dass die Konzentration an während der Reaktion gebildetem Wasser und/oder Alkohol so gesteuert wird, dass sie unter 150 mM gehalten wird.

EP 1 426 445 A1

## Beschreibung

### Gebiet der Erfindung

Die Erfindung befindet sich auf dem Gebiet der Phyto- und Biochemie und betrifft ein Verfahren zur enzymatischen Herstellung von Flavonoidderivaten, die im Lebensmittelbereich, sowie in der Kosmetik und in pharmazeutischen Zubereitungen eingesetzt werden.

### Stand der Technik

Die biologischen Wirkungen der Flavonoide sind seit vielen Jahren gut bekannt. Indem sie verschiedene oxidierende Spezies abfangen, verhindern sie eine oxidative Schädigung von Biomolekülen wie DNA, Lipiden und Proteinen. In Antioxidans-Assays sind einige Flavonoide wirksamer als die Vitamine C und E. Neben dieser Haupteigenschaft wurden mehrere weitere biologische Wirkungen aufgezeigt, einschließlich der Inhibierung der Wirkung von Enzymen und der Proliferation von Tierzellen, Viren und Bakterien. Weiterhin haben sie eine Wirkung auf das Gefäßsystem und eine starke antioxidative Kapazität.

Wegen ihrer hautschützenden und -reinigenden Eigenschaften und ihrer Wirkungen gegen Altern, gegen Hautverfärbungen und auf das Aussehen der Haut wurden Flavonoide auch als Bestandteile von kosmetischen bzw. dermopharmazeutischen Zusammensetzungen. Weiterhin wirken sie auf die mechanischen Eigenschaften des Haars.

Die Antioxidationseigenschaften der Flavonoide hängen von der Molekülstruktur ab. Untersuchungen zur Struktur-Wirkungs-Beziehung haben gezeigt, dass die antioxidative Wirkung auf einer ortho-Hydroxylierung am Ring B des Moleküls, der Anzahl freier Hydroxylgruppen, dem Vorhandensein einer Doppelbindung zwischen Kohlenstoff 2 und 3 im Ring C und dem Vorhandensein einer Hydroxylgruppe an Kohlenstoff 3 (Abbildung I ) beruht.

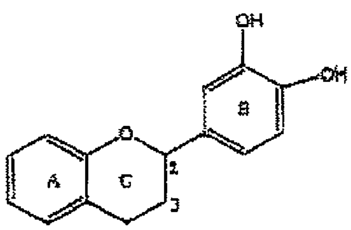

(I)

Die Verwendung dieser Moleküle wird prinzipiell einerseits durch eine sehr geringe Löslichkeit in wäßrigen wie auch organischen Medien und andererseits durch eine geringe Stabilität eingeschränkt. Flavonoide werden durch Licht, Sauerstoff bzw. Oxidationsmittel und Temperaturerhöhungen abgebaut. Diese Einschränkungen verhindern ihren wirksamen Einsatz in Lebensmitteln und kosmetischen und pharmazeutischen Zusammensetzungen.

Zur Behebung dieses Stabilitätsproblems gibt es verschiedene gut bekannte Strategien: eine Einkapselung oder eine Formulierung mit Antioxidationsmitteln. Keine dieser Lösungen ist jedoch vollständig zufriedenstellend, und es besteht weiterhin ein Bedarf an neuen glycosylierten Flavonoiden und Aglyconflavonoiden mit erhöhter Stabilität.

Zur Verbesserung der Eigenschaften dieser Moleküle wurden enzymatische und chemische Modifikationen vorgeschlagen. So wird in den Patentschriften JP55157580 und JP58131911 die Acylierung von Quercetin mit Fettsäurechloriden in Dioxan in Gegenwart von Pyridin erwähnt. In diesen Patenten wird die Acylierung jedoch in Gegenwart toxischer Lösungsmittel durchgeführt. Die Substratumwandlungsraten sind niedrig. Die Acylierung von Flavon, Flavonol und Flavanon auf gleiche Weise wurde in den Coletica-Patenten FR 2778663 (US6235294) beschrieben. Diese Umsetzung wurde chemisch in Gegenwart eines Fettsäurechlorids oder -anhydrids durchgeführt. Die Durchführung dieser Reaktionen erfordert die Verwendung der aktivierten Fettsäuren und toxischer Lösungsmittel (Pyridin, Chloroform und Toluol). Weiterhin wurden hohe Temperaturen (100°C) verwendet, und die Substratumwandlungsraten waren gering (etwa 10 bis 60%). Außerdem sind diese Reaktionen nicht selektiv, was zu polyacylierten Produkten führt. Im Patent WO 099660 wird die chemische Acylierung von Flavonoiden (Quercetin, Galangin, (+)-Catechin) durch Fettsäuren (Laurylsäure, Buttersäure, Essigsäure...) und einen anschließenden enzymatischen Hydrolyseschritt durch eine Lipase von *Mucor miehei* erwähnt. Diese Erfindung weist die gleichen Nachteile wie das Patent FR2778663 (US6235294) auf. Nach der zunächst durchgeführten Acylierungsreaktion sind die gebildeten Produkte polyacyliert. Sind Monoester gewünscht, so ist in einem zweiten Schritt eine enzymatische Hydrolyse erforderlich, die nach dem Entfernen der Lösungsmittel der ersten Umsetzung zur Vermeidung einer Desaktivierung des Enzyms durchgeführt wird. Flavonoidmodifikationen wurden auch im Patent EP0618203 beschrieben, in dem die Acylierung von (+/-)-Catechin durch Essigsäureethylester und Propionsäureethylester und die Acylierung von Epigallocatechin durch Propionsäure- und Buttersäurephenylester erwähnt werden. Zur Durchführung dieser Reaktion benötigt man ein teures Enzym (die Carboxylase von *Streptomyces rochei*), und die Umwandlungsraten der beiden Substrate ist sehr niedrig (weniger als 1% des Acyldonors). Schließlich wird im Henkel/Cognis-Patent WO 0179245 eine enzymatische Acylierung von Flavonoiden (Naringin, Rutin, Asparatin, Orientin, Quercetin, Kämpferol, cis-Orientin, Isoquercitrin) durch verschiedene Säuren (*p*-Chlorphenylessigsäure, Stearinsäure, 12-Hydroxystearinsäure, Palmitinsäure, Laurinsäure, Caprinsäure, 4-Hydroxyphenylessigsäure, 5-Phenylvaleriansäure, Cumarsäure, Ölsäure, Linolsäure) beschrieben. In diesem Patent wird ein Verfahren beschrieben, bei dem eine hohe Konzentration an *Candida antarctica* (40 g/l) und, bezogen auf die Flavonoide, ein Überschuß an Acyldonor verwendet wird. Die Umwandlungsrate der Substrate ist niedrig (10 bis 20%).

Alle oben beschriebenen Arbeiten sind durch niedrige Ausbeuten gekennzeichnet. Darüber hinaus werden bei den chemischen Modifikationen toxische Lösungsmittel verwendet, die Reaktionen sind unspezifisch, was zu Produktmischungen führt, und die beschriebenen enzymatischen Reaktionen sind auf glycosylierte Flavonoide beschränkt. Die im Stand der Technik erwähnten niedrigen Umwandlungsausbeuten beruhen auf der Tatsache, dass die eingesetzten Verfahren nicht an Reaktionen mit wenig löslichen Substraten angepaßt sind und durch das während der Umsetzung entstehende Wasser in drastischer Weise beeinträchtigt werden.

**Beschreibung der Erfindung**

Gegenstand der Erfindung ist ein Verfahren zur enzymatischen Synthese von Flavonoidestern und -derivaten, bei dem man

a) ein Reaktionsmedium herstellt, enthaltend ein organisches Lösungsmittel, ein glycosyliertes Flavonoid oder Aglyconflavonoid, einen Acylgruppendonor und einen enzymatischen Katalysator,
b) während der Reaktion weitere Mengen an Flavonoid und/oder Acyldonor zusetzt und
c) die so erhaltenen Ester reinigt, indem man enzymatische Partikel und das Lösungsmittel abtrennt,

dadurch charakterisiert, dass die Konzentration an während der Reaktion gebildetem Wasser und/oder Alkohol so gesteuert wird, dass sie unter 150 mM gehalten wird.

Die vorliegende Erfindung - ein Verfahren zur selektiven Acylierung von glycosylierten Flavonoiden und Aglyconflavonoiden - führt zur Verbesserung der Flavonoidderivate bezüglich ihrer Stabilität und Löslichkeit in verschiedenen Zubereitungen, wobei ihre antioxidativen Eigenschaften erhalten bleiben bzw. verbessert werden. Ein weiterer besonderer Vorteil, der durch diese modifizierten Flavonoide erzielt wird, ist, dass bifunktionelle Moleküle mit höherer biologischer Wirksamkeit gebildet werden.

Verglichen mit den aus dem Stand der Technik bekannten Methoden läßt sich mit diesem Verfahren eine deutliche Verbesserung in bezug auf die Endkonzentrationen von Flavonoidestern, die Umwandlungsausbeuten (sowohl für die Flavonoide als auch den ursprünglich vorhandenen Acyldonor) und insbesondere die Produktivität erzielen, während die komplizierten, aufwendigen Aufreinigungs-operationen nach der Synthese reduziert werden.

Bei diesem Verfahren kommt eine Enzymtechnologie unter milden Temperatur- und Druckbedingungen zum Einsatz, ohne dass dabei irgendwelche gefährlichen Lösungsmittel verwendet werden, wobei die Flavonoidester durch direkte Veresterung oder Umestern gemäß den folgenden Reaktionsschemata gebildet werden:

$$\text{Flavonoid} + \text{RCOOH} \rightarrow \text{Flavonoid-OCOR} + H_2O$$

$$\text{Flavonoid} + \text{RCOOR'} \rightarrow \text{Flavonoid-OCOR} + \text{R'OH}$$

wobei R' für eine C1-C4-Alkylgruppe, vorzugsweise eine C1-C2-Alkylgruppe, steht.

Dieses Verfahren ist dadurch gekennzeichnet, dass zunächst das Reaktionsmedium entwässert wird, um vor Beginn der Reaktion vorhandenes Wasser zu entfernen, und das bzw. der während der Reaktion gebildete Wasser bzw. Alkohol on-line entfernt wird. Wasser und/oder Alkohol werden auf Konzentrationen gehalten, die für die verwendeten Lösungsmittel und Substrate geeignet sind, vorzugsweise auf Konzentrationen von unter 150 mM, besonders bevorzugt unter 100 mM.

Das Verfahren eignet sich für eine Vielzahl von Aglyconflavonoiden und glycosylierten Flavonoiden, und die mit diesem milden enzymatischen Verfahren erzielten Umwandlungsausbeuten liegen über den bislang erhaltenen: im Bereich von 50 bis 99%.

Die enzymatische Synthese wird unter milderen Bedingungen als die chemischen Synthesen durchgeführt,

wobei der Einsatz von toxischen Lösungsmitteln wie Pyridin, Benzol und THF, hohe Temperaturen und das Anfallen von Nebenprodukten wie Salzen oder Flavonoid-Abbauprodukten, die zusätzliche Reinigungsschritte erfordern würden, vermieden wird.

Verglichen mit den obenerwähnten bekannten Methoden läßt sich mit diesem Verfahren eine deutliche Verbesserung in bezug auf die Endkonzentrationen von Flavonoidestern, die Umwandlungsausbeuten (sowohl für die Flavonoide als auch den ursprünglich vorhandenen Acyldonor) und insbesondere die Produktivität erzielen, während die komplizierten, aufwendigen Aufreinigungsoperationen nach der Synthese reduziert werden.

Ein Unterschied zwischen dem erfindungsgemäßen Verfahren und den bekannten Methoden des Standes der Technik ist die Art, in der die enzymatische Reaktion durchgeführt wird, was beträchtlich höhere Ausbeuten ermöglicht, und die Vielzahl verschiedener Flavonoide, die eingesetzt werden können (sowohl glycosylierte Formen als auch Aglyconformen).

Das Hauptziel der Erfindung ist es, alle der obenerwähnten Nachteile bestehender Acylierungsmethoden zu reduzieren und ein Verfahren zur enzymatischen Synthese von Flavonoidestern vorzuschlagen, das, verglichen mit den obenerwähnten bekannten Methoden, eine deutliche Verbesserung in bezug auf die Endkonzentrationen von Flavonoidestern, die Umwandlungsausbeuten (sowohl für die Flavonoide als auch den ursprünglich vorhandenen Acyldonor) und insbesondere die Produktivität erlaubt, während die komplizierten, aufwendigen Aufreinigungsoperationen nach der Synthese reduziert werden.

Hierzu betrifft die Erfindung ein Verfahren zur enzymatischen Synthese von Flavonoidestern, das dadurch gekennzeichnet ist, dass man in einen hierfür ausgelegten Reaktor zur Bildung eines Reaktionsmediums vorbestimmte Mengen eines Flavonoids (glycosylierte Formen und Aglyconformen) oder Flavonoidderivats, eines Acylgruppendonors, eines organischen Lösungsmittels, bei dem es sich um den Acyldonor handeln kann, und eines enzymatischen Katalysators einbringt, unter Bedingungen, bei denen zunächst das Reaktionsmedium auf eine Wasserkonzentration von unter 150 mM, vorzugsweise unter 100 mM getrocknet werden kann und bei der die Konzentration an während der Reaktion gebildetem Wasser und/oder Alkohol unter einem vorgegebenen Punkt von 150 mM, vorzugsweise 100 mM gehalten werden kann. Dieser vorgegebene Punkt wird durch on-line Entfernen des gebildeten Wassers und/oder Alkohols durch Adsorption an Molekularsieben, durch Destillation oder durch Pervaporation eingehalten. Diese Reaktion läßt sich im Batch-Verfahren oder auch im Fed-Batch-Verfahren mit einem oder mehreren Substraten durchführen. Beim Fed-Batch-Verfahren läßt sich das Molverhältnis vom Flavonoid zum Acyldonor durch ein geeignetes Substratzugabeprofil während der Reaktion konstant halten. Es ist somit möglich zu steuern, wie sich die Zusammensetzung des Reaktionsmediums im Verlauf der Zeit entwickelt, und somit die enzymatische Reaktion in Richtung auf eine maximale Produktion an mono- oder multiacylierten Verbindungen zu lenken und gleichzeitig störende Reaktionen zu begrenzen. Die so erhaltenen Flavonoidester werden abschließend gereinigt, indem man wenigstens enzymatische Partikel (beispielsweise durch Dekantieren, Filtrieren oder Zentrifugieren) und das Lösungsmittel (beispielsweise durch Abdampfen, Destillieren oder Membranfiltration) abtrennt.

Die Umsetzung wird erfindungsgemäß so durchgeführt, dass die Inhibierung bzw.

Desaktivierung der Enzymreaktion, die man in Gegenwart hoher Konzentrationen an Flavonoiden, Acyldonoren oder Wasseransammlungen beobachtet, zunächst eingeschränkt ist. Die Substrate können im Verlauf der Reaktion graduell in kontrollierter Weise zugeführt werden, wodurch vermieden wird, dass Konzentrationen erreicht werden, die die Enzymreaktion hemmen würden.

Die Reaktion kann so durchgeführt werden, dass das Flavonoid:Acyldonor-Molverhältnis von 0,01 bis 20,00, vorzugsweise von 0,02 bis 10,00, beträgt. Indem man die Werte für das Molverhältnis im Reaktionsmedium in den oben angegebenen Bereichen hält, ist es möglich, entweder höhere Reaktionsgeschwindigkeiten oder maximale Anteile an monoacylierten Flavonoiden zu erzielen. Durch Steuerung von Art und Quantität der im Verlauf der Zeit zugegebenen Reagentien läßt sich das Molverhältnis während der Reaktion konstant halten oder kontrolliert variieren, so dass es im Verlauf der Zeit ein definiertes Variationsprofil durchläuft, sich aber dennoch während der gesamten Reaktion in dem obenerwähnten Wertebereich befindet. Eine Optimierung der Synthesereaktion ist durch zwischenzeitliches oder kontinuierliches Abziehen wenigstens eines Bestandteils des Reaktionsmediums möglich. Der abgezogene Bestandteil/die abgezogenen Bestandteile können möglicherweise nach Fraktionierung in den Reaktor zurückgeführt werden.

Gemäß einer Ausführungsform der Erfindung kann vorgesehen sein, zwischenzeitlich oder kontinuierlich das gesamte Reaktionsmedium abzuziehen und nach Fraktionierung einen oder mehrere Bestandteile des abgezogenen Mediums wieder in den Reaktor zu injizieren.

Das zur Durchführung des erfindungsgemäßen Verfahrens verwendete Reaktionsgefäß bzw. der hierfür verwendete Reaktor ist vorzugsweise mit Vorrichtungen zur Steuerung der Temperatur, des Wasser- und/oder Alkoholgehalts und des Drucks, mit Vorrichtungen zur Zugabe von Reagentien und mit Vorrichtungen zum Abziehen von Produkten versehen.

Während des Ablaufs der Synthesereaktion wird die Temperatur vorteilhafterweise auf 20 - 100°C und der Partialdruck über dem Reaktionsmedium vorteilhafterweise auf 10 mbar ($10^3$ Pa) bis 1000 mbar ($10^5$ Pa) eingestellt,

ausgehend von einem auf eine Konzentration von unter 150 mM, vorzugsweise unter 100 mM eingestellten Ausgangswassergehalt, die Menge an Wasser und/oder Alkohol wird auf unter 150 mM, vorzugsweise unter 100 mM gehalten und das Reaktionsmedium wird vorteilhafterweise leicht gerührt.

Zum Erhalt von Zubereitungen aus hochreinen Flavonoidestern kann weiterhin vorgesehen sein, zusätzliche abschließende Fraktionierungsoperationen durchzuführen, beispielsweise indem man die verbliebenen Flavonoide oder Fette durch Extraktion mit organischen Lösungsmitteln oder superkritischen Fluiden, durch Destillation bzw. molekulare Destillation, durch Ausfällen oder durch Kristallisieren abtrennt.

Bei dem in der Erfindung verwendeten Aglyconflavonoid oder glycosylierten Flavonoid bzw. Flavonoidderivat kann es sich um eine beliebige aus der von Chalcon, Flavon, Flavanol, Anthocyan und Flavanon, Flavonol, Cumarin, Isoflavonen und Xanthonen gebildeten Gruppe ausgewählte Verbindung handeln.

Die Acyldonorverbindung wird unter bekannten Fettsäuren bzw. deren Methyl-, Ethyl-, Propyl- oder Butylestern ausgewählt. Diese Fettsäure wird vorzugsweise aus der von einer geradkettigen oder verzweigten aliphatischen Säure, gesättigt, ungesättigt oder cyclisch, mit bis zu 22 Kohlenstoffatomen, gegebenenfalls substituiert durch einen oder mehrere Substituenten ausgewählt aus der aus Hydroxyl, Amino, Mercapto, Halogen und Alkyl-S-S-alkyl bestehenden Gruppe, beispielsweise Palmitinsäure, 16-Hydroxyhexadecansäure, 12-Hydroxystearinsäure, 11-Mercaptoundecansäure, Thioctansäure oder Chinasäure, geradkettigen oder verzweigten aliphatischen Disäuren, gesättigt oder ungesättigt, mit bis zu 22 Kohlenstoffatomen, beispielsweise Hexadecandisäure oder Azelainsäure, einer arylaliphatischen Säure und einer davon abgeleiteten dimeren Säure, einer Zimtsäure, gegebenenfalls substituiert durch einen oder mehrere Substituenten ausgewählt aus der aus Hydroxyl, Nitro, Alkyl, Alkoxy und Halogenatomen bestehenden Gruppe, beispielsweise Kaffeesäure (3,4-Dihydroxyzimtsäure), Ferulasäure (4-Hydroxy-3-methoxyzimtsäure) oder Cumarsäure (4-Hydroxyzimtsäure), einer Benzoesäure, gegebenenfalls substituiert durch einen oder mehrere Substituenten ausgewählt aus der Gruppe Hydroxyl, Nitro, Alkyl, Alkoxy und Halogenatomen, beispielsweise Gallussäure (3,4,5-Trihydroxybenzoesäure), Vanillinsäure (4-Hydroxy-3-methoxybenzoesäure) oder Protocatechusäure (3,4-Dihydroxybenzoesäure) gebildeten Gruppe ausgewählt. Die Fettsäureester werden vorzugsweise aus den Methyl- oder Ethylestern der obigen Verbindungen ausgewählt.

Die Reaktion läßt sich mit dem Acyldonor als Lösungsmittel oder in einem geeigneten Lösungsmittel, bei dem es sich um eine beliebige organische Verbindung oder eine beliebige Mischung organischer Verbindungen handeln kann, in der die ausgewählten Flavonoide bzw. Flavonoidderivate und Acyldonoren ganz oder teilweise solubilisiert werden, durchführen. So wird das/die Lösungsmittel insbesondere aus den folgenden Substanzen ausgewählt: Propan-2-ol, Butan-2-ol, Isobutanol, Aceton, Propanon, Butanon, Pentan-2-on, 1,2-Ethandiol, 2,3-Butandiol, Dioxan, Acetonitril, 2-Methylbutan-2-ol, *tert*.-Butanol, 2-Methylpropanol und 4-Hydroxy-2-methylpentanon, aliphatischen Kohlenwasserstoffen wie Heptan, Hexan oder einer Mischung von zwei oder mehreren dieser Lösungsmittel.

Der verwendete enzymatische Katalysator muß natürlich den Transfer einer Acylgruppe von einem Acyldonor zu einem Flavonoid bzw. Flavonoidderivat bewirken und fördern und ist vorteilhafterweise eine Protease oder Lipase, z.B. aus *Candida antarctica, Rhizomucor miehei, Candida cylindracea, Rhizopus arrhizus*, vorzugsweise auf einem Träger immobilisiert.

Im Betracht der verschiedenen obenerwähnten Merkmale kann man sich mehrere verschiedene Ausführungsformen der Erfindung vorstellen, insbesondere in Hinsicht auf die Art der verwendeten Reagentien und die bevorzugten zu erreichenden Ziele.

In einer ersten Ausführungsform kann man sich somit ein Syntheseverfahren in einem Batch-Reaktor vorstellen (beide Substrate werden in den Reaktor mit Lösungsmittel und Enzym gegeben). In diesem Fall enthält der Reaktor zunächst das Lösungsmittel, die Gesamtmenge an Flavonoiden (im allgemeinen von 1 g/l bis 200 g/l), die erforderlich ist, um die gewünschte Endmenge an modifizierten Flavonoiden zu erhalten, und die Menge an freier Säure als Acyldonor, die dem ursprünglich erforderlichen Molverhältnis (von gelöstem Flavonoid/Acyldonor) (im allgemeinen von 0,01 bis 20) entspricht. Um einen festgelegten Punkt der Menge an Wasser im Reaktor von unter 100 mM zu erzielen, wird das Medium unter Vakuum (10-500 mbar, vorzugsweise 50-250 mbar) auf eine Temperatur von 20-100°C, vorzugsweise 40-80°C, erhitzt, und die erzeugte Dampfmischung wird in einer mir Molekularsieben gefüllten Säule getrocknet und anschließend kondensiert und in den Reaktor zurückgeführt. Falls erforderlich wird das Kondensat über eine zweite mit Molekularsieben gefüllte Säule zurückgeführt. Dann wird das Enzym in löslicher oder immobilisierter Form (von 1 g/l bis 100 g/l, vorzugsweise von 5 g/l bis 20 g/l) zugesetzt. Während der Reaktion gebildetes Wasser wird über die mit Molekularsieben gefüllte Säule entfernt, indem man das Vakuum und die Temperatur im Reaktor entsprechend einstellt.

In einer zweiten Ausführungsform kann man sich somit ein Syntheseverfahren vorstellen, bei dem Acyldonor und Lösungsmittel während der Umsetzung zugegeben werden. In diesem Fall enthält der Reaktor zunächst das Lösungsmittel, die Gesamtmenge an Flavonoiden (im allgemeinen von 1 g/l bis 200 g/l), die erforderlich ist, um die gewünschte Endmenge an modifizierten Flavonoiden zu erhalten, und die Menge an freier Säure als Acyldonor, die dem ursprünglich erforderlichen Molverhältnis (von gelöstem Flavonoid/Acyldonor) (im allgemeinen von 0,01 bis 20) entspricht. Um einen festgelegten Punkt der Menge an Wasser im Reaktor von unter 100 mM zu erzielen, wird das Medium

unter Vakuum (10-500 mbar, vorzugsweise 50-250 mbar) auf eine Temperatur von 20-100°C, vorzugsweise 40-80°C, erhitzt, und die erzeugte Dampfmischung wird in einer mit Molekularsieben gefüllten Säule getrocknet und anschließend kondensiert und in den Reaktor zurückgeführt. Falls erforderlich wird das Kondensat über eine zweite mit Molekularsieben gefüllte Säule zurückgeführt. Dann wird das Enzym in löslicher oder immobilisierter Form (von 1 g/l bis 100 g/l, vorzugsweise von 5 g/l bis 20 g/l) zugesetzt. Während des Ablaufs der Reaktion wird Lösungsmittel zugesetzt, so dass ein Teil des Lösungsmittels über eine Säule mit Molekularsieben abgedampft wird. Das Wasser wird durch Austausch in der Dampfphase entfernt. Der Dampf wird kondensiert und in einem Auffangbehälter gesammelt.
Um die Lösungsmittelmenge relativ konstant zu halten, führt man während der Reaktion on-line Acyldonor in einer solchen Menge pro Zeiteinheit zu, dass das Molverhältnis (von gelöstem Flavonoid/Acyldonor) auf dem erforderlichen Wert gehalten wird. Somit wird, wenn es vorteilhaft ist, dieses Molverhältnis während der Reaktion konstant zu halten, der Acyldonor mit einer Geschwindigkeit zugesetzt, die der Geschwindigkeit seines Verbrauchs in der Umsetzung entspricht; diese Geschwindigkeit des Verbrauchs läßt sich durch eine vorbereitende kinetische Untersuchung der eingesetzten Enzymreaktion bestimmen. Die während der Reaktion pro Zeiteinheit zugesetzte Menge an Acyldonor beläuft sich im allgemeinen auf von 0,01 bis 10 Gramm Acyldonor pro Stunde pro Gramm Enzymkatalysator im Reaktor.

In einer dritten Ausführungsform der Erfindung kann das Syntheseverfahren auch unter Zugabe von Flavonoiden und Lösungsmittel durchgeführt werden. In diesem Fall enthält der Reaktor zunächst das Lösungsmittel, die Gesamtmenge an freier Säure als Acyldonor (im allgemeinen von 1 g/l bis 500 g/l), die erforderlich ist, um die gewünschte Endmenge an modifizierten Flavonoiden zu erhalten, und die Menge an Flavonoid, die dem ursprünglich erforderlichen Molverhältnis (von gelöstem Flavonoid/Acyldonor) (im allgemeinen von 1 g/l bis 200 g/l) entspricht. Um einen festgelegten Punkt der Menge an Wasser im Reaktor von unter 100 mM zu erzielen, wird das Medium unter Vakuum (10-500 mbar, vorzugsweise 50-250 mbar) auf eine Temperatur von 20-100°C, vorzugsweise 40-80°C, erhitzt, und die erzeugte Dampfmischung wird in einer mit Molekularsieben gefüllten Säule getrocknet und anschließend kondensiert und in den Reaktor zurückgeführt. Falls erforderlich wird das Kondensat über eine zweite mit Molekularsieben gefüllte Säule zurückgeführt. Dann wird das Enzym in löslicher oder immobilisierter Form (von 1 g/l bis 100 g/l, vorzugsweise von 5 g/l bis 20 g/l) zugesetzt.
Während des Ablaufs der Umsetzung setzt man Lösungsmittel zu und legt ein Vakuum an, so dass ein Teil des Lösungsmittels und das gebildete Wasser abgedampft werden. Die Verdampfungsmenge wird durch eine entsprechende Steuerung des Vakuums und der Temperatur eingestellt. Die gebildeten Dämpfe werden über eine mit Molekularsieben gefüllte Säule geführt. Durch den Kontakt mit den Molekularsieben wird das Wasser entfernt. Nach dem Entfernen des Wassers wird der Dampf kondensiert und in einem Auffangbehälter gesammelt, um dann später in den Reaktor zurückgeführt zu werden. Gegebenenfalls führt man während der Reaktion wasserfreies Lösungsmittel zum Ausgleich von Verdampfungsverlusten zu und um die Lösungsmittelmenge relativ konstant zu halten. Weiterhin wird Flavonoid in einer solchen Menge pro Zeiteinheit zugesetzt, dass das Molverhältnis (von gelöstem Flavonoid/Acyldonor) auf dem erforderlichen Wert gehalten wird. Somit wird, wenn es vorteilhaft ist, dieses Molverhältnis während der Reaktion konstant zu halten, das Flavonoid mit einer Geschwindigkeit zugesetzt, die der Geschwindigkeit seines Verbrauchs in der Umsetzung entspricht; diese Geschwindigkeit des Verbrauchs läßt sich durch eine vorbereitende kinetische Untersuchung der eingesetzten Enzymreaktion bestimmen. Die während der Reaktion pro Zeiteinheit zugesetzte Menge an Flavonoid beläuft sich im allgemeinen auf von 0,01 bis 10 Gramm Flavonoid pro Stunde pro Gramm Enzymkatalysator im Reaktor.

In einer vierten Ausführungsform der Erfindung kann das Syntheseverfahren auch unter Zugabe von Flavonoid, Acyldonor und Lösungsmittel durchgeführt werden. In diesem vierten Fall enthält der Reaktor zunächst das Lösungsmittel, eine variable Konzentration an Flavonoid (vorzugsweise höher als die Löslichkeit des Flavonoids im Lösungsmittel) und die Menge an freier Säure als Acyldonor, die dem ursprünglich erforderlichen Molverhältnis (von gelöstem Flavonoid/Acyldonor) entspricht. Um einen festgelegten Punkt der Menge an Wasser im Reaktor von unter 100 mM zu erzielen, wird das Medium unter Vakuum (10-500 mbar, vorzugsweise 50-250 mbar) auf eine Temperatur von 20-100°C, vorzugsweise 40-80°C, erhitzt, und die erzeugte Dampfmischung wird in einer mit Molekularsieben gefüllten Säule getrocknet und anschließend kondensiert und in den Reaktor zurückgeführt. Falls erforderlich wird das Kondensat über eine zweite mit Molekularsieben gefüllte Säule zurückgeführt. Dann wird das Enzym in löslicher oder immobilisierter Form (von 1 g/l bis 100 g/l, vorzugsweise von 5 g/l bis 20 g/l) zugesetzt. Während des Ablaufs der Umsetzung setzt man Lösungsmittel zu und legt ein Vakuum im Bereich von 10-500 mbar, vorzugsweise 100-250 mbar, an. Zum Entfernen des Wassers werden die gebildeten Dämpfe über eine Säule mit Molekularsieben geführt. Der Dampf wird kondensiert und in einem Auffangbehälter gesammelt. Gegebenenfalls führt man während der Reaktion wasserfreies Lösungsmittel zum Ausgleich von Verdampfungsverlusten zu und um die Lösungsmittelmenge relativ konstant zu halten. Weiterhin wird Flavonoid in einer solchen Menge pro Zeiteinheit zugesetzt, dass das Molverhältnis (von gelöstem Flavonoid/Acyldonor) auf dem erforderlichen Wert gehalten wird.
Ist es vorteilhaft, dieses Molverhältnis während der Reaktion konstant zu halten, so setzt man Flavonoid und Acyldonor in Mengen pro Zeiteinheit zu, die jeweils der Geschwindigkeit ihres Verbrauchs in der Umsetzung entsprechen; diese Verbrauchsgeschwindigkeiten lassen sich durch eine vorbereitende kinetische Untersuchung der eingesetzten Enzymreaktion bestimmen.

In einer fünften Ausführungsform der Erfindung kann das kontinuierliche Syntheseverfahren alternativ unter Zugabe und Abziehen von Flavonoid, Acyldonor und/oder Lösungsmittel und möglicherweise Enzymkatalysator durchgeführt werden. In diesem fünften Fall enthält der Reaktor zunächst das Lösungsmittel, eine variable Konzentration an Flavonoid (vorzugsweise höher als die Löslichkeit des Flavonoids im Lösungsmittel) und die Menge an freier Säure als Acyldonor, die dem ursprünglich erforderlichen Molverhältnis (von gelöstem Flavonoid/Acyldonor) entspricht. Um einen festgelegten Punkt der Menge an Wasser im Reaktor von unter 100 mM zu erzielen, wird das Medium unter Vakuum (10-500 mbar, vorzugsweise 50-250 mbar) auf eine Temperatur von 20-100°C, vorzugsweise 40-80°C, erhitzt, und die erzeugte Dampfmischung wird in einer mit Molekularsieben gefüllten Säule getrocknet und anschließend kondensiert und in den Reaktor zurückgeführt. Falls erforderlich wird das Kondensat über eine zweite mit Molekularsieben gefüllte Säule zurückgeführt. Dann wird das Enzym in löslicher oder immobilisierter Form zugesetzt. Während die Umsetzung stattfindet, werden kontinuierlich oder zwischenzeitlich Substanzen aus dem Reaktionsmedium abgezogen. Liegt das Enzym in immobilisierter Form vor, so kann es im Reaktor zurückgehalten werden. Nach der Trennung kann man das Lösungsmittel und möglicherweise das Flavonoid und/oder den Acyldonor in den Reaktor zurückführen. Während der Reaktion wird wasserfreies Lösungsmittel zugesetzt, so dass Verluste durch Verdampfen und Abziehen ausgeglichen werden, und es ist weiterhin möglich, Flavonoid und Acyldonor zuzusetzen, in solchen Mengen pro Zeiteinheit, dass das Molverhältnis dieser beiden Bestandteile auf dem erforderlichen Wert gehalten wird. Das Wasser wird wie oben beschrieben durch Molekularsiebe entfernt. Nach dem Entwässern wird das verdampfte Lösungsmittel kondensiert und wieder in den Reaktor zurückgeführt. Ist es vorteilhaft, dieses Molverhältnis während der Reaktion konstant zu halten, so werden Flavonoid und Acyldonor in Mengen pro Zeiteinheit zugesetzt, die ihren jeweiligen Verbrauchsgeschwindigkeiten in der Umsetzung und ihren jeweiligen Abzugsgeschwindigkeiten entsprechen.

Bei einer sechsten Ausführungsform führt man die Umsetzung wie oben durch, ersetzt jedoch die freie Säure als Acyldonor durch ihren Methyl-, Ethyl-, Propyl- oder Butylester, vorzugsweise ihren Methyl- oder Ethylester. Der gebildete Alkohol wird auf die gleiche Weise wie oben entfernt.

In einer siebten Ausführungsform wird der Acyldonor als Lösungsmittel verwendet.

In einer achten Ausführungsform wird im Medium vorhandenes bzw. vorhandener und/oder während der Umsetzung gebildetes bzw. gebildeter Wasser und/oder Alkohol durch eine Pervaporationsmembran entfernt, in der Dampf- oder Flüssigphase.

## Beispiele

### Beispiel 1:

Die Rutinmonopalmitatsynthese wurde in einem 250-ml-Batch-Reaktor unter Verwendung von *Candida antarctica*-Lipase (Novozym 435) durchgeführt. Hierbei handelt es sich um eine auf einem makroporösen Acrylharz immobilisierte Lipase. Die Lipase wird mit einer Aktivität von 7000 PLE x $g^{-1}$ (Propyllauratsynthese), einem Wassergehalt von 1-2 Gew.-% und einem enzymatischen Proteingehalt im Bereich zwischen 1 und 10 Gew.-% geliefert.

Für diese Synthese wurden 0,75 g (1,2 mmol) Rutin, 0,315 g (1,2 mmol) Palmitinsäure und 250 ml *tert*.-Amylalkohol verwendet. Das Medium wurde unter Vakuum (150 mbar) auf 60°C erhitzt, und der gebildete Dampf wurde über eine auf 60°C erhitzte und mit 50 g Molekularsieben gefüllte Säule geleitet. Das vorhandene Wasser wurde also in der Gasphase entfernt, was sehr viel effektiver ist als in der Flüssigphase. Der entwässerte Dampf wurde kondensiert und über eine zweite Säule, die mit der gleichen Menge an Molekularsieben gefüllt war, in den Reaktor zurückgeführt. Durch diese Vorgehensweise war es möglich, nach 6 h einen Ausgangswassergehalt von unter 100 mM zu erhalten und die Substrate zu solubilisieren. Dann wurde das Enzym (2,5 g) zugesetzt. Die Umsetzung wurde bei 60°C unter Vakuum (150 mbar) durchgeführt, und das gebildete Wasser wurde auf die gleiche Weise wie zur ursprünglichen Trocknung entfernt, wodurch dessen Konzentration auf unter 100 mM gehalten wurde.

Diese Konzentration läßt sich durch eine entsprechende Einstellung des Vakuums und Kühlen des Kühlers variieren. Die untersuchten Drücke schwanken zwischen 10 und 700 mbar, und die Temperatur des Kühlers zwischen -20 und 5°C. Durch diese Vorgehensweise ist es möglich, die Wasserkonzentration im Reaktor auf zwischen 10 und 400 mM einzustellen.

Nach 48 Stunden Umsetzung zeigte die Produktanalyse durch HPLC, dass die Umwandlungsausbeuten für die beiden Substrate etwa 90% betrugen.

Am Ende der Reaktion wurde das Enzym durch Filtrieren wiedergewonnen. Das Medium wurde dann durch Abdampfen des Lösungsmittels konzentriert. Zum Eliminieren von Substratresten wurden zwei Extraktionssysteme eingesetzt. Zum Entfernen der Palmitinsäure wurde eine Mischung von Acetonitril/Heptan (3/5, v/v) verwendet, während das Rutin durch Extraktion mit Wasser/Heptan (2/3, v/v) abgetrennt wurde.

Die Struktur des Produkts wurde durch $^1$H-NMR-Analyse bestätigt.
$^1$H-NMR: (400 MHz, DMSO-$d_6$): δ 0,8 (t, 3H), 1 (d, 3H), 1,25 (m, 24H), 1,45 (m, 2H), 2,1 (m, 2H), 3,1-3,6 (breit, C-H

Zucker), 3,7 (d, 1H), 4,45 (s, 1H), 4,65 (t, 1H), 5,3 (breit, OH Zucker), 5,1 (breit, OH Zucker), 5,45 (d, 1 H), 6,2 (s, 1 H), 6,4 (s, 1H), 6,8 (d, 1H), 7,6 (m, 2H), 12,6 (s, 1H, $C_5$-OH) ppm

**Beispiel 2:**

Die Acylierung von Hesperidin (0,75 g, 1,2 mmol) mit Palmitinsäure (0,315 g, 1,2 mmol) wurde wie oben beschrieben durchgeführt.

Die HPLC-Analyse zeigte, dass nach 48 Stunden 95% des Acyldonors verbraucht waren. Durch die gleiche Aufreinigungsvorschrift wie oben konnte mittels Flüssig-Flüssig-Extraktion das Hesperidinmonopalmitat gewonnen werden. Die Struktur dieses Hesperidinesters wurde durch $^1$H-NMR-Analyse bestätigt.
$^1$H-NMR: (400 MHz, DMSO-$d_6$): δ 0,83 (t, 3H), 1,0 (d, 3H), 1,05 (breit, 24H), 1,20 (m, 2H), 2,25 (m, 2H), 3,4-3,6 (breit, C-H Zucker), 3,8 (s, 3H), 4,15 (s, 1H), 4,58 (s, 1H), 4,75 (m, 2H), 5,0 (m, 1H), 5,18 (dd, 1H), 5,4 (d, 1H), 5,48 (d, 1H), 6,14 (m, 1H), 6,18 (s, 1H), 7,0 (m, 3H), 9,15 (s, 1H), 12,05 (s, 1H) ppm

**Beispiel 3:**

Die Acylierung von Esculin (0,75 g, 2 mmol) mit Palmitinsäure (0,523 g, 2 mmol) wurde wie in Beispiel 1 beschrieben durchgeführt.

Die Flüssigchromatographieanalyse zeigte, dass nach 48 Stunden 80% des Acyldonors verbraucht waren. Durch die gleiche Aufreinigungsvorschrift wie oben konnte mittels Flüssig-Flüssig-Extraktion das Esculinmonopalmitat gewonnen werden. Die Struktur dieses Esculinesters wurde durch $^1$H-NMR-Analyse bestätigt.
$^1$H-NMR: (400 MHz, DMSO-$d_6$): δ 0,8 (t, 3H), 1,15 (breit, 24H), 1,4 (m, 2H), 2,25 (m, 2H), 3,2 (m, 1H), 3,65 (m, 1H), 4,1 (dd, 1H), 4,35 (d, 1H), 4,85 (d, 1H), 5,25 (s, 1H), 5,35 (d, 1H), 6,2 (d, 1 H), 6,8 (s, 1H), 7,3 (s, 1H), 7,85 (d, 1 H) ppm

**Beispiel 4:**

Die Acylierung von Quercetin (0,75 g, 2,2 mmol) mit Palmitinsäure (0,568 g, 2,2 mmol) wurde wie in Beispiel 1 beschrieben durchgeführt.

Die Analyse durch Flüssigchromatographie zeigte, dass nach 72 Stunden jeweils 50% der beiden Substrate verbraucht waren.

## Patentansprüche

1. Verfahren zur enzymatischen Synthese von Flavonoidestern und -derivaten, bei dem man

   a) ein Reaktionsmedium herstellt, enthaltend ein organisches Lösungsmittel, ein glycosyliertes Flavonoid oder Aglyconflavonoid, einen Acylgruppendonor und einen enzymatischen Katalysator,
   b) während der Reaktion weitere Mengen an Flavonoid und/oder Acyldonor zusetzt und
   c) die so erhaltenen Ester reinigt, indem man enzymatische Partikel und das Lösungsmittel abtrennt,

   dadurch charakterisiert, dass die Konzentration an während der Reaktion gebildetem Wasser und/oder Alkohol so gesteuert wird, dass sie unter 150 mM gehalten wird.

2. Verfahren nach Anspruch 1, dadurch charakterisiert, dass die Konzentration an während der Reaktion gebildetem Wasser und/oder Alkohol so gesteuert wird, dass sie unter 100 mM gehalten wird.

3. Verfahren nach Anspruch 1, dadurch charakterisiert, dass das Molverhältnis von Flavonoid zu Acyldonor im Reaktionsmedium so eingestellt ist, dass es während des Verfahrens im Bereich von 0,01 bis 20,00 liegt.

4. Verfahren nach Anspruch 1, dadurch charakterisiert, dass das Molverhältnis von Flavonoid zu Acyldonor im Reaktionsmedium so eingestellt ist, dass es während des Verfahrens im Bereich von 0,02 bis 10,00 liegt.

5. Verfahren nach Anspruch 1, bei dem man weiterhin dem Reaktionsmedium kontinuierlich oder zwischenzeitlich weitere Mengen an wenigstens einem Flavonoid in fester Form oder in Form einer flüssigen Lösung, Lösungsmittel, enzymatischem Katalysator in löslicher oder immobilisierter Form und Azyldonorverbindung alleine oder solubilisiert im Lösungsmittel zusetzt.

**6.** Verfahren nach Anspruch 1, bei dem man weiterhin zwischenzeitlich oder kontinuierlich wenigstens einen Bestandteil des Reaktionsmediums abzieht und diesen wenigstens einen abgezogenen Bestandteil nach Fraktionierung wieder in den Reaktor zurückführt.

**7.** Verfahren nach Anspruch 6, bei dem man weiterhin zwischenzeitlich oder kontinuierlich das gesamte Reaktionsmedium abzieht und nach Fraktionierung einen oder mehrere Bestandteile des abgezogenen Mediums wieder in den Reaktor injiziert.

**8.** Verfahren nach Anspruch 1, wobei während der Reaktion die Temperatur auf 20-100°C eingestellt wird, der Partialdruck über dem Reaktionsmedium auf 10 mbar bis 1000 mbar eingestellt wird und das Reaktionsmedium gerührt wird.

**9.** Verfahren nach Anspruch 1, bei dem man weiterhin verbliebene Flavonoide oder verbliebenen Acyldonor durch Extraktion mit organischen Lösungsmitteln oder superkritischen Fluiden, durch Destillation, durch Kristallisation, durch Adsorption oder durch Ausfällen eliminiert.

**10.** Verfahren nach Anspruch 1, bei dem man weiterhin die dargestellten Flavonoidester durch Ausfällen oder chromatographische Trennung fraktioniert.

**11.** Verfahren nach Anspruch 1, bei dem das Flavonoid ausgewählt ist aus der Gruppe, die gebildet wird von Chalcon, Flavon, Flavonol, Flavanon, Anthocyan, Flavanol, Cumarin, Isoflavon und Xanthon.

**12.** Verfahren nach Anspruch 1, wobei die Acyldonorverbindung ausgewählt ist aus der Gruppe, die gebildet wird von geradkettigen oder verzweigten aliphatischen Säuren, gesättigt, ungesättigt oder cyclisch, mit bis zu 22 Kohlenstoffatomen, gegebenenfalls substituiert durch einen oder mehrere Substituenten ausgewählt aus der aus Hydroxyl, Amino, Mercapto, Halogen und Alkyl-S-S-alkyl bestehenden Gruppe, beispielsweise Palmitinsäure, 16-Hydroxyhexadecansäure, 12-Hydroxystearinsäure, 11-Mercaptoundecansäure, Thioctansäure oder Chinasäure, geradkettigen oder verzweigten aliphatischen Disäuren, gesättigt oder ungesättigt, mit bis zu 22 Kohlenstoffatomen, beispielsweise Hexadecandisäure ode Azelainsäure, einer arylaliphatischen Säure und einer davon abgeleiteten dimeren Säure, einer Zimtsäure, gegebenenfalls substituiert durch einen oder mehrere Substituenten ausgewählt aus der aus Hydroxyl, Nitro, Alkyl, Alkoxy und Halogenatomen bestehenden Gruppe, beispielsweise Kaffeesäure (3,4-Dihydroxyzimtsäure), Ferulasäure (4-Hydroxy-3-methoxyzimtsäure) oder Cumarsäure (4-Hydroxyzimtsäure), einer Benzoesäure, gegebenenfalls substituiert durch einen oder mehrere Substituenten ausgewählt aus der Gruppe Hydroxyl, Nitro, Alkyl, Alkoxy und Halogenatomen, beispielsweise Gallussäure (3,4,5-Trihydroxybenzoesäure), Vanillinsäure (4-Hydroxy-3-methoxybenzoesäure) oder Protocatechusäure (3,4-Dihydroxybenzoesäure), oder deren Methyl-, Ethyl-, Propyl- oder Butylestern.

**13.** Verfahren nach Anspruch 1, wobei das organische Lösungsmittel ausgewählt ist aus der Gruppe, die gebildet wird von Propan-2-ol, Butan-2-ol, Isobutanol, Aceton, Propanon, Butanon, Pentan-2-on, 1,2-Ethandiol, 2,3-Butandiol, Dioxan, Acetonitril, 2-Methylbutan-2-ol, *tert*.-Butanol, 2-Methylpropanol und 4-Hydroxy-2-methylpentanon, aliphatischen Kohlenwasserstoffen wie Heptan und Hexan und Mischungen aus mindestens zwei dieser Komponenten.

**14.** Verfahren nach Anspruch 1, wobei es sich bei dem organischen Lösungsmittel um den Acyldonor handelt.

**15.** Verfahren nach Anspruch 1, wobei der enzymatische Katalysator eine Protease und/oder Lipase umfaßt.

**16.** Verfahren nach Anspruch 15, wobei die Protease und/oder Lipase auf einem Träger immobilisiert ist.

**17.** Verfahren nach Anspruch 1, wobei das Wasser und/oder der Alkohol in der Gasoder Flüssigphase durch Molekularsiebe aus dem Medium entfernt wird.

**18.** Verfahren nach Anspruch 1, wobei das Wasser und/oder der Alkohol in der Gasoder Flüssigphase durch Pervaporation aus dem Medium entfernt wird.

Europäisches Patentamt

# EUROPÄISCHER RECHERCHENBERICHT

Nummer der Anmeldung
EP 02 29 2969

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| A | GAO C ET AL: "NOVEL ENZYMATIC APPROACH TO THE SYNTHESIS OF FLAVONOID GLYCOSIDES AND THEIR ESTERS" BIOTECHNOLOGY AND BIOENGINEERING - COMBINATORIAL CHEMISTRY, WILEY, NEW YORK, NY, US, Bd. 71, Nr. 3, 2001, Seiten 235-243, XP001010601 ISSN: 0006-3592 * das ganze Dokument * --- | 1-18 | C12P7/62 C12P17/06 |
| A | DANIELI ET AL: "Enzyme-mediated regioselective acylations of flavonoid disaccharide monoglycosides" HELVETICA CHIMICA ACTA, VERLAG HELVETICA CHIMICA ACTA. BASEL, CH, Bd. 73, Nr. 7, 1990, Seiten 1837-1844, XP002090940 ISSN: 0018-019X * das ganze Dokument * --- | 1-18 | |
| A,D | WO 01 79245 A (HENKEL KGAA (DE); ) 25. Oktober 2001 (2001-10-25) * das ganze Dokument * --- | 1-18 | RECHERCHIERTE SACHGEBIETE (Int.Cl.7) C12P |
| A,D | US 6 235 294 B1 (MARIOTTE ANNE-MARIE ET AL) 22. Mai 2001 (2001-05-22) * das ganze Dokument * ----- | 1-18 | |

Der vorliegende Recherchenbericht wurde für alle Patentansprüche erstellt

| Recherchenort | Abschlußdatum der Recherche | Prüfer |
|---|---|---|
| MÜNCHEN | 29. April 2003 | Douschan, K |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR. EP 02 29 2969

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

29-04-2003

| Im Recherchenbericht angeführtes Patentdokument | Datum der Veröffentlichung | Mitglied(er) der Patentfamilie | Datum der Veröffentlichung |
|---|---|---|---|
| WO 0179245 A | 25-10-2001 | DE 10019235 A1 | 31-10-2001 |
| | | AU 5042301 A | 30-10-2001 |
| | | WO 0179245 A1 | 25-10-2001 |
| | | EP 1274712 A1 | 15-01-2003 |
| US 6235294 B1 | 22-05-2001 | FR 2778663 A1 | 19-11-1999 |
| | | DE 19922287 A1 | 25-11-1999 |
| | | JP 2000026263 A | 25-01-2000 |
| | | US 2001031735 A1 | 18-10-2001 |

EPO FORM P0461

Für nähere Einzelheiten zu diesem Anhang : siehe Amtsblatt des Europäischen Patentamts, Nr.12/82